# EUROPEAN PATENT APPLICATION

(11) **EP 4 137 494 A1**
(43) Date of publication of application: **22.02.2023**
(21) Application number: 20931031.7
(22) Date of filing: 17.04.2020
(51) Int. Cl.: C07D 495/04, A61K 31/5025, A61P 35/00

(54) **CRYSTAL FORM OF THIOFURAN PYRIDAZINE COMPOUND, PREPARATION METHOD THEREFOR AND APPLICATION THEREOF**

(71) Applicant: ZheJiang Medicine Co., Ltd. Xinchang Pharmaceutical Factory, Shaoxing, Zhejiang 312500 (CN); Zhejiang Changhai Pharmaceutical Co., Ltd., Shaoxing, Zhejiang 312366 (CN)
(72) Inventor: SHEN, Dadong, Shaoxing, Zhejiang 312500 (CN); LV, Yonghui, Shaoxing, Zhejiang 312500 (CN); WU, Guofeng, Shaoxing, Zhejiang 312500 (CN); ZHANG, Dingfeng, Shaoxing, Zhejiang 312500 (CN); CAO, Ruiwei, Shaoxing, Zhejiang 312500 (CN)
(74) Representative: Murgitroyd & Company
(86) International application number: PCT/CN2020/085268
(87) International publication number: WO 2021/208055

(57) **Abstract**

The present invention provides a crystal form of a thiofuran pyridazine compound, a preparation method therefor and an application thereof. The crystal form of the thiofuran pyridazine compound is (S)-2-(4-fluorophenyl)-4-(piperidine-3-aminomethyl)thieno[2,3-d] pyridazine-7-carboxamide hydrochloride of formula (I). The crystal form of the thiofuran pyridazine compound in the present invention has higher stability and is stored especially in high humidity conditions, and thus is suitable for preparing various forms of drugs.

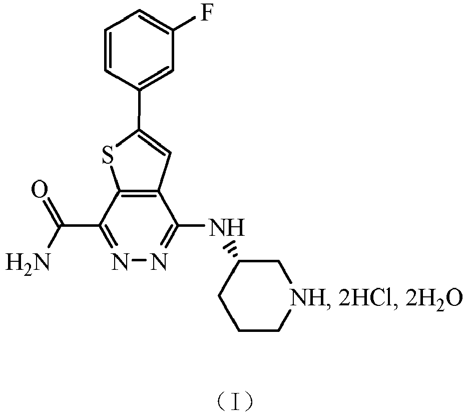

## Description

### FIELD OF THE INVENTION

The present invention relates to crystal form of (S)-2-(4-fluorophenyl)-4-(piperidine-3-aminomethyl)thieno[2,3-d]pyridazine-7-carboxamide hydrochloride and preparation method therefor and application thereof.

### BACKGROUND OF THE INVENTION

(S)-2-(4-fluorophenyl)-4-(piperidine-3-aminomethyl)thieno[2,3-d]pyridazine-7-carboxa mide is a potent inhibitor of the kinase CHK1, thus has the ability to prevent cell cycle arrest at G2/M checkpoint in response to DNA damage. Consequently, these compounds have anti-cell proliferative (*e.g.* anti-cancer) activity and can also be used in combination with other anti-tumor drugs so as to enhance curative effects of other anti-tumor drugs. Therefore, it can be used for the treatment of human or animals.

### SUMMARY OF THE INVENTION

The technical problem to be solved by the present invention is to provide a crystal form of a thiofuran pyridazine compound with high stability under high humidity storage conditions, so as to be suitable for preparing stable pharmaceutical preparations.

For this purpose, the present invention adopts the technical solution as follows: a crystal form of a thiofuran pyridazine compound, in particular, relates to (S)-2-(4-fluorophenyl)-4-(piperidine-3-aminomethyl)thieno[2,3-d]pyridinazine-7-carboxamide hydrochloride, its structural formula (I) is as follows:

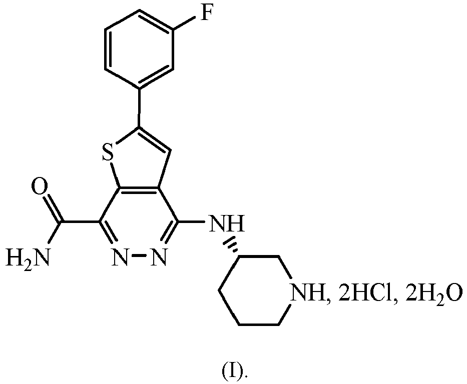

X-ray powder diffraction expressed by a 2θ diffraction angle (instrument model: Brucker D8 Advance X-ray powder diffractometer; the test conditions: 3.000°-40.005°, Step0.020°, Step time: 0.4s, Anode: Cu, 25°C), the pattern shows the strongest and second strongest characteristic diffraction peaks at 4.11±0.20°, 5.82±0.20°, 6.85±0.20°, 7.41±0.20°, 8.22±0.20°, 9.67±0.20°, 13.73±0.20°, 14.10±0.20°, 14.85±0.20°.

The purpose of the present invention is to provide a preparation method of the above-mentioned new crystal form, comprisimg the steps as follows: (S)-2-(4-fluorophenyl)-4-(piperidine-3-aminomethyl)thieno[2,3-d]pyridazine-7-carboxamide is salified with hydrochloric acid in an aqueous medium at a temperature of 10-100°C, to obtain crystals and then separated and dried to a constant weight to remove adsorbed water and solvent.

The amount of water and hydrochloric acid is sufficient to form a stoichiometric hydrochloride hydrate, and is sufficient to thoroughly mix raw materials with water and hydrochloric acid. It may use any amount of water because it would occur the absorption of crystal water in the process of salt formation of raw materials to lead to the formation of hydrates, and no further hydrates will be obtained. An appropriate amount of water should be limited to allow complete mixing and only has a low solubility loss.

The separated compound crystals may be dried at room temperature or at a higher temperature (less than 70°C), under normal pressure or reduced pressure.

The isolation of the compound of the present invention may be carried out at 0-60°C, preferably at 10-30°C. The aqueous medium may be (C₁∼C₃) alkanol, such as methanol, ethanol, n-propanol, isopropanol, etc., or a solvent to be soluble with water, such as acetone, butanone, acetonitrile, 1,4-dioxane or a mixed solvent to be soluble with water.

The present invention has the following beneficial effects: the crystal form has higher stability, especially may be stored under high humidity conditions, and may be suitable for preparing medicines in various forms.

### DESCRIPTION OF DRAWINGS

FIG.1 is shows an X-ray powder diffraction pattern of the compound of the present invention.
FIG.2 shows a TGA spectrum of the compound of the present invention.
FIG.3 shows a DSC spectrum of the compound of the present invention.
FIG.4 is shows an infrared spectrum of the compound of the present invention.

### DETAILED DESCRIPTION AND PREFERRED EMBODIMENTS THEREOF

Hereafter, the present invention will be described specifically with reference to the examples and figures. The examples are given only for illustration of the technical solution of the present invention and should not be construed to limit the present invention. The experimental methods not specified in the following embodiments shall be selected according to conventional methods and conditions, or according to the instructions. All percentages and servings are calculated by weight unless otherwise stated.

### Example 1:

To dissolve 1g of (S)-2-(4-fluorophenyl)-4-(piperidine-3-aminomethyl)thieno[2,3-d] pyridazine-7-carboxamide to 10ml of solvent of water: methanol (1:1), and add 0.2ml of hydrochloric acid, and then heat up to reflux untill dissolve to clarify, afterwards cool to precipitate crystals, filter, and then dry under reduced pressure, to obtain 0.96g of product with a purity of 99.12%.

### Example 2:

To dissolve 1g of (S)-2-(4-fluorophenyl)-4-(piperidine-3-aminomethyl)thieno[2,3-d] pyridazine-7-carboxamide to 10ml of solvent of water: ethanol (3:7), and add 0.2ml of hydrochloric acid, and then heat up to reflux untill dissolve to clarify, afterwards cool to precipitate crystals, filter, and then dry under reduced pressure, to obtain 0.90g of product with a purity of 99.30%.

### Example 3:

To dissolve 1g of (S)-2-(4-fluorophenyl)-4-(piperidine-3-aminomethyl)thieno[2,3-d] pyridazine-7-carboxamide to 10ml of solvent of water: isopropanol (2:8), and add 0.2ml of hydrochloric acid, and then heat up to reflux untill dissolve to clarify, afterwards cool to precipitate crystals, filter, and then dry under reduced pressure, to obtain 0.92g of product with a purity of 99.21%.

### Example 4:

To dissolve 1g of (S)-2-(4-fluorophenyl)-4-(piperidine-3-aminomethyl)thieno[2,3-d] pyridazine-7-carboxamide to 10ml of solvent of water: acetone (3:7), and add 0.2ml of hydrochloric acid, and then heat up to reflux untill dissolve to clarify, afterwards cool to precipitate crystals, filter, and then dry under reduced pressure, to obtain 0.88g of product with a purity of 99.68%.

### Example 5:

To dissolve 1g of (S)-2-(4-fluorophenyl)-4-(piperidine-3-aminomethyl)thieno[2,3-d] pyridazine-7-carboxamide to 10ml of solvent of water: acetonitrile (3:7), and add 0.2ml of hydrochloric acid, and then heat up to reflux untill dissolve to clarify, afterwards cool to precipitate crystals, filter, and then dry under reduced pressure, to obtain 0.89g of product with a purity of 99.74%.

### Example 6: Performance Test Experiment

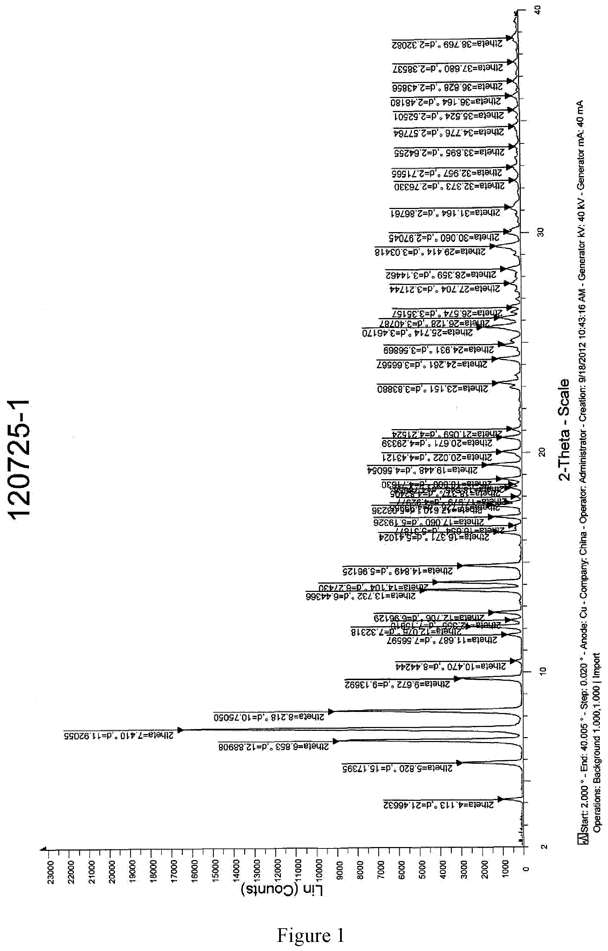

XRD, TGA and DSC experiments were carried out on the products obtained from the above examples. It can be seen from the experiments that the X-ray powder diffraction patterns (XRD), TGA and DSC patterns of the products obtained from the above examples are consistent with FIG.1, FIG.2, FIG.3 and FIG.4 of the drawings of the description.

Wherein, use the Brucker D8 Advance X-ray powder diffractometer for X-ray powder diffraction, and the test conditions: 3.000⁰-40.005⁰, Step0.020⁰, Step time 0.4s, Anode:Cu, 25°C. TGA instrument model: TA Q500, DSC instrument model: TA Q2000. 10°C/min program heating. Infrared instrument model: Nicolet iS10 FI-IR, KBr tablets.

As shown in FIG.1, FIG.1 shows the X-ray powder diffraction pattern of the compound of the present invention. According to the X-ray powder diffraction pattern datum in FIG.1, the sample shows the strongest and second strongest characteristic diffraction peaks at 4.11 ±0.20° , 5.82±0.20° , 6.85±0.20° , 7.41 ±0.20° , 8.22±0.20° , 9.67±0.20° , 13.73±0.20° , 14.10±0.20° , 14.85±0.20° .

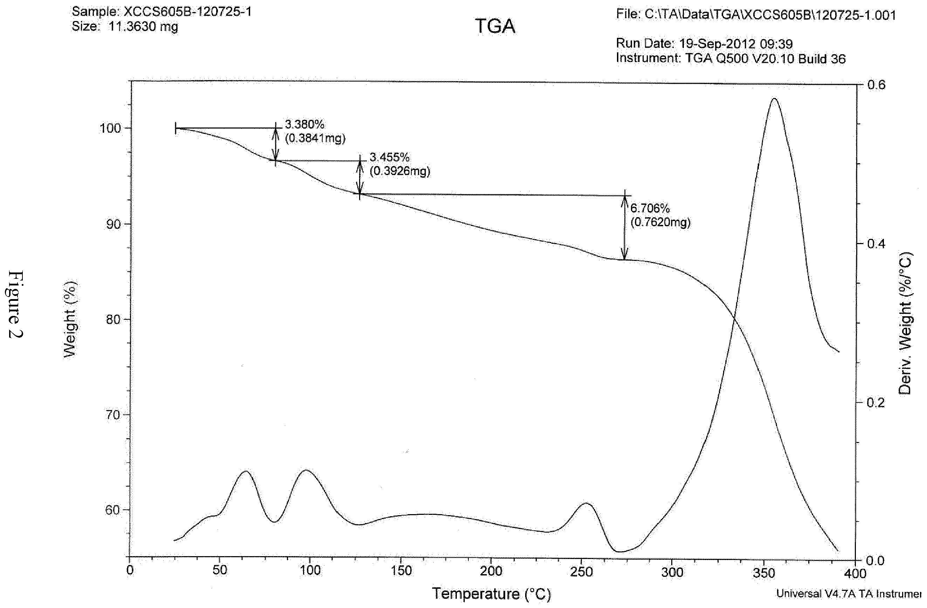

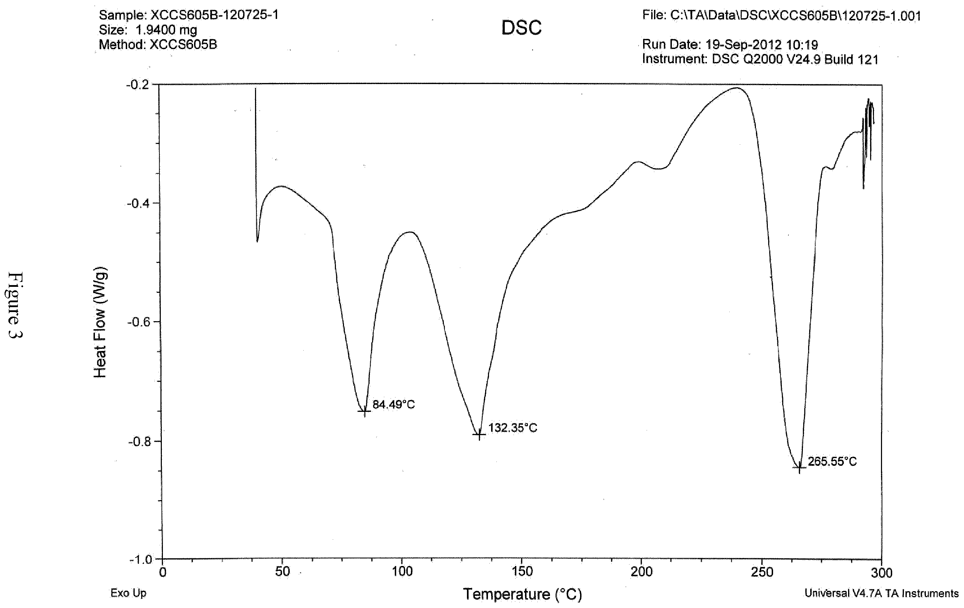

As shown in FIG.2 and FIG.3, FIG.2 shows the TGA spectrum of the compound of the present invention, and FIG.3 shows the DSC spectrum of the compound of the present invention. According to the TGA spectrum and DSC spectrum datum in FIG.2 and FIG.3: (1) TGA analysis shows that the sample is a hydrate, TGA analysis shows that the sample loses two molecules of crystal water when the temperature reaches about 120°C; the sample loses about one molecule of hydrogen chloride when the temperature reaches about 260°C; and then continue to increase the temperature, the sample decomposed. (2) DSC analysis shows that the sample has an endothermic peak at 84°C, 132°C respectively, the endothermic peak correspondings to the loss of two molecules of crystalline water of the sample respectively. There is an endothermic peak at about 255 °C, which is caused by the melting decomposition of the sample.

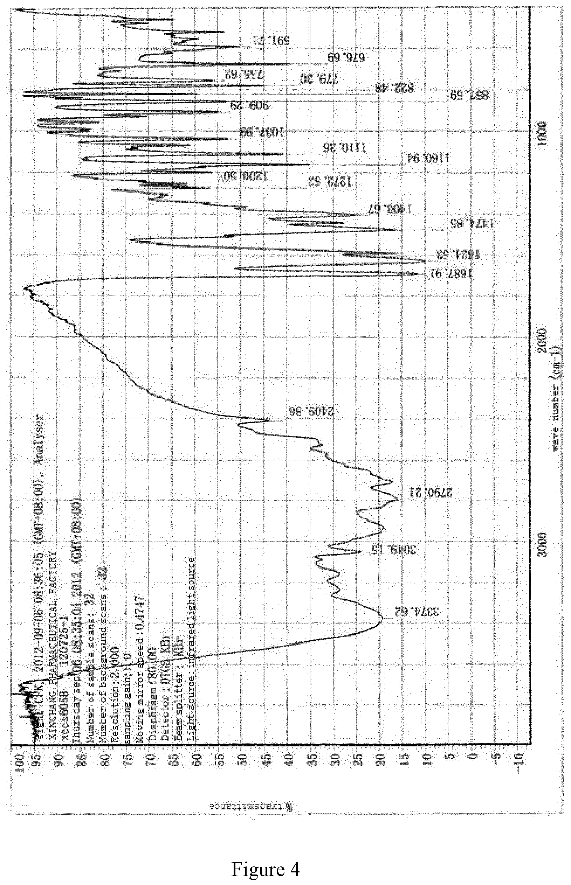

As shown in FIG.4, FIG.4 shows the infrared spectrum of the compound of the present invention. According to the infrared spectrum datum in FIG.4, the sample shows characteristic absorption peaks at 3374.62 cm-1, 3049.15 cm-1, 2790.21 cm-1, 1624.53 cm-1, 1474.85 cm-1, 1403.67 cm-1, 200.50 cm-1, 1160.94 cm-1, 822.48 cm-1 and 779.30 cm-1.

### Example 7: Stability Test

Stability tests were performed on the samples of Examples 1, 2, 3, 4 and 5. The datum of acceleration test (40°C±2°C, relative humidity 75%±5%) and long-term stability test show that the crystal of the samples is stability and there is no significant changes in moisture, related substances, optical isomers and contents under storage conditions for 36 months (temperature 25°C±2°C; relative humidity 60%±5%). The datum of the acceleration test show that the crystal of the samples is stability showed and there is no significant changes in moisture, related substances, optical isomers and contents under storage conditions for 6 months (temperature 40°C ±2°C; relative humidity 75%±5%). Please refer to the results of the acceleration test (40°C±2°C, relative humidity 75%±5%) of Table 1 and the results of the long-term stability test (25°C±2°C, relative humidity 60%±10%) of Table 2.

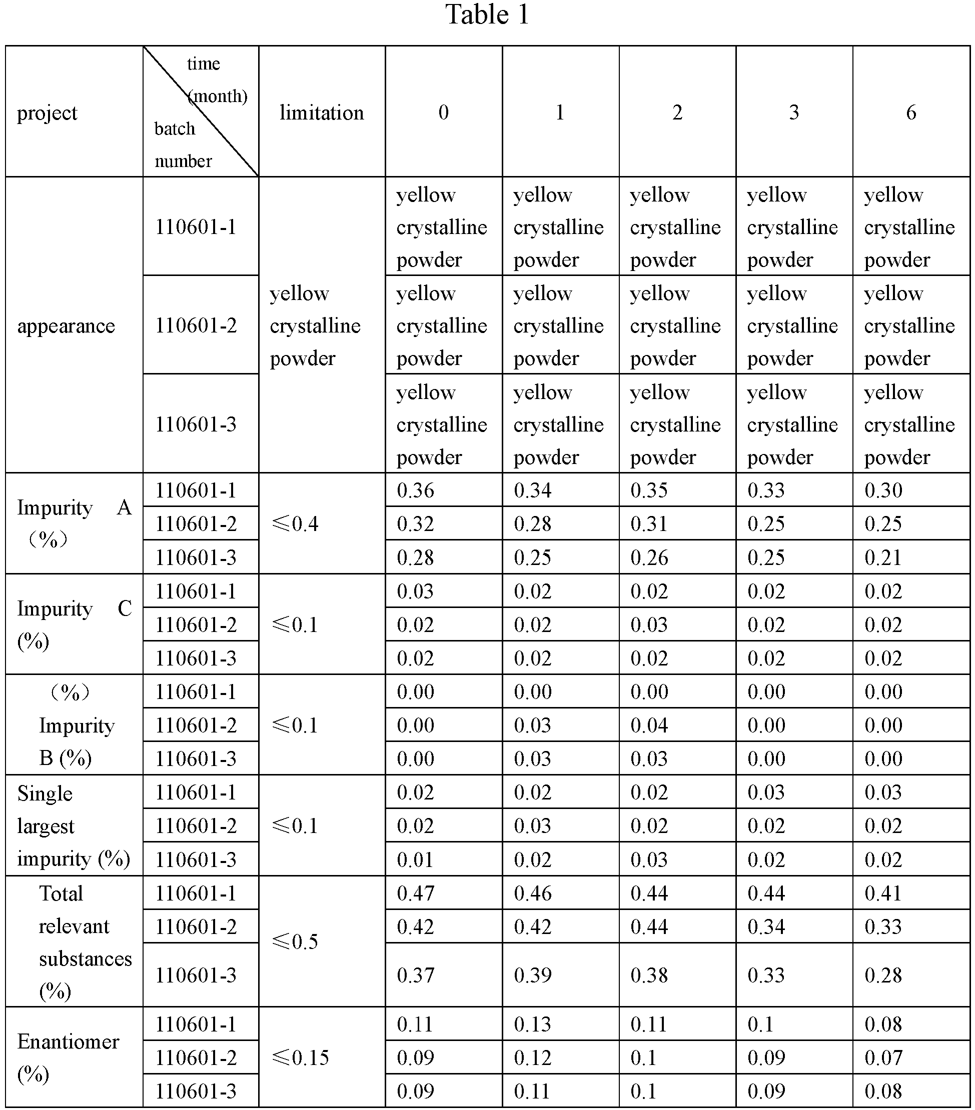

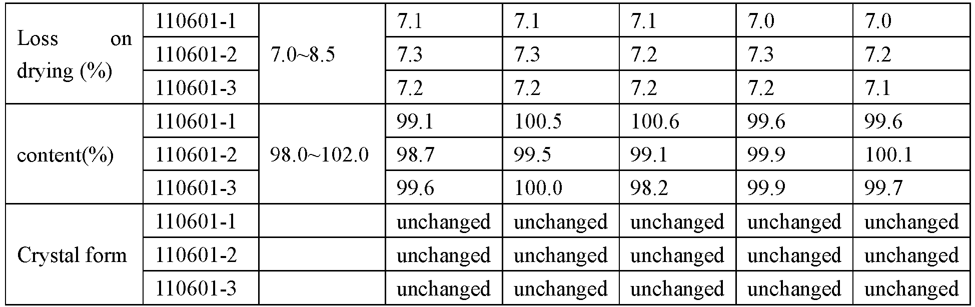

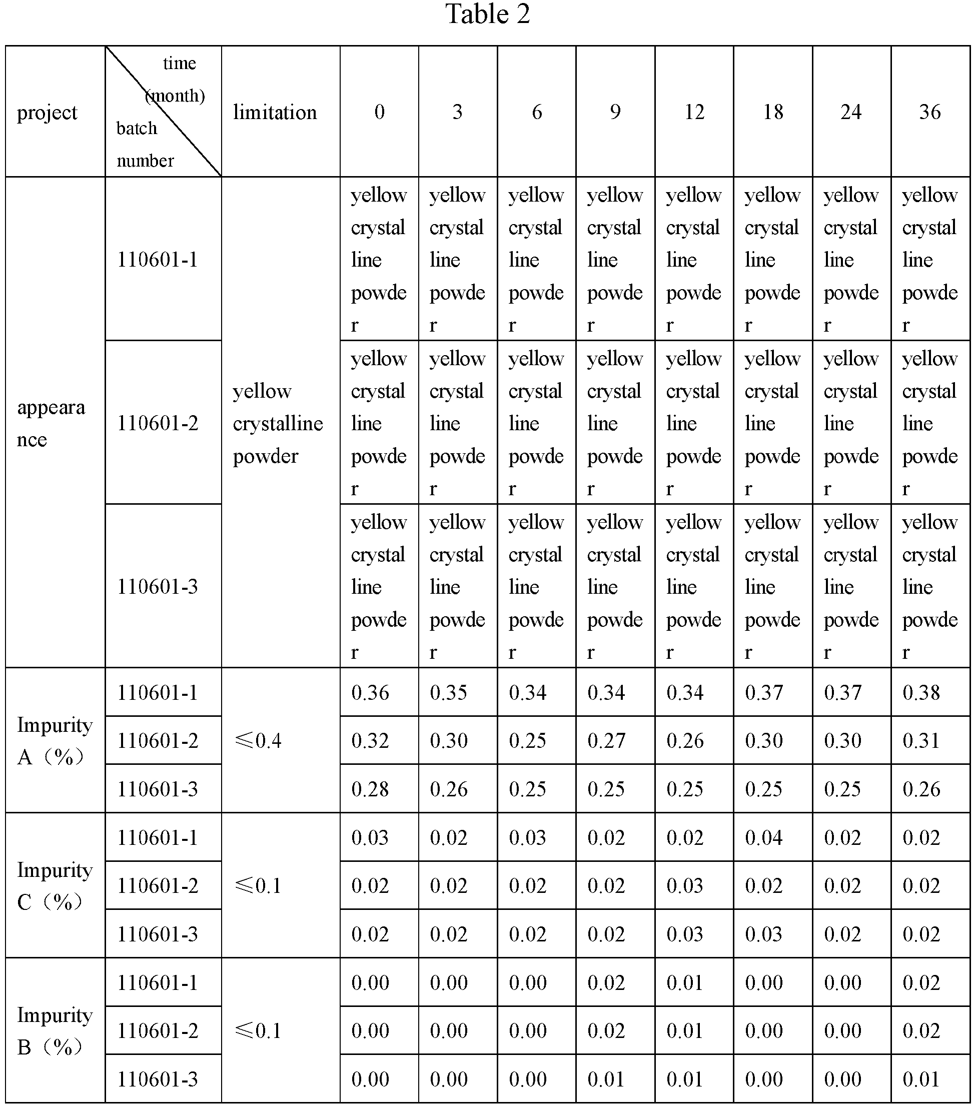

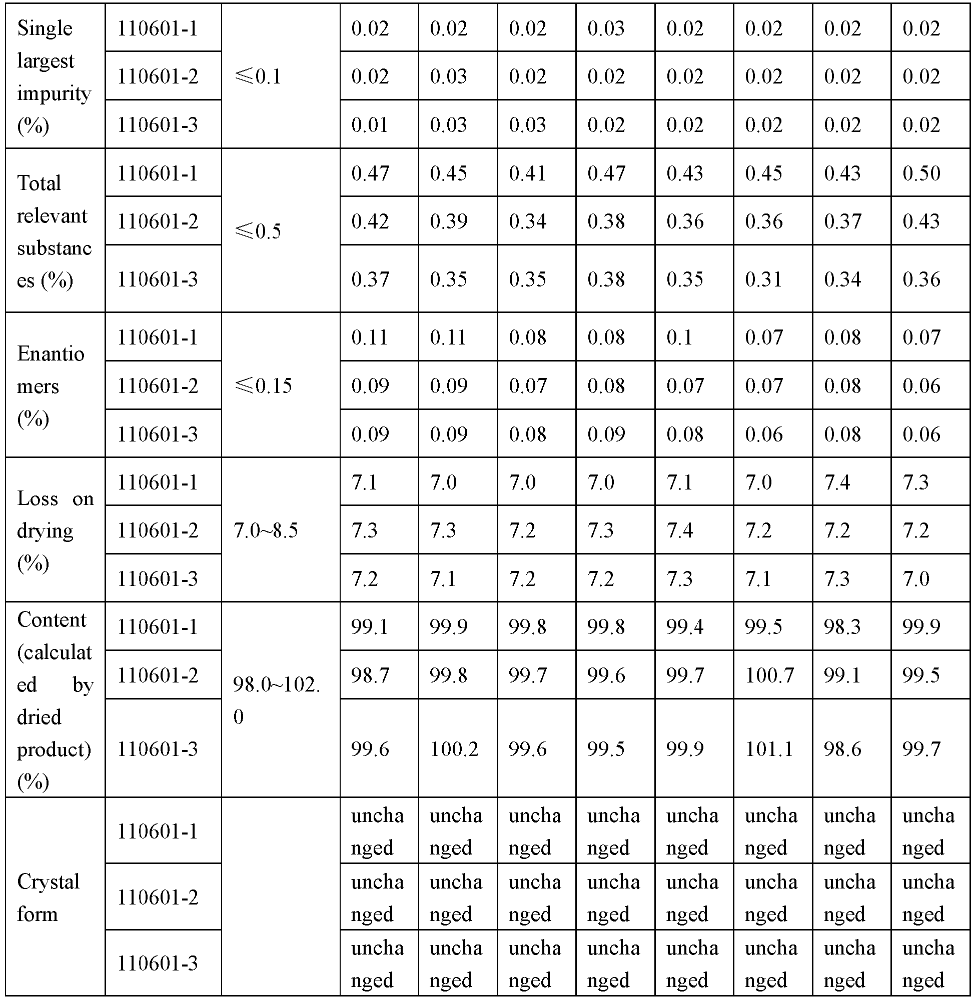

The present invention illustrates by the above invention summary and examples, however, the present invention is not limited to special instance and implementation scheme described herein. It is easy for any persons skilled in the art to carry out further improvement and perfection not from the spirit and scope of the invention, so the present invention is just limited by the content and scope of claims of the present invention, its intention to cover all included all alternative solutions and equivalent solutions within the spirit and scope of the present invention limited by the appendix claims.

## Claims

1. A crystal form of thiofuran pyridazine compound, the crystal form of thiofuran pyridazine compound is (S)-2-(4-fluorophenyl)-4-(piperidine-3-aminomethyl)thieno[2,3-d] pyridinazine-7-carboxamide hydrochloride as shown in formula (I),

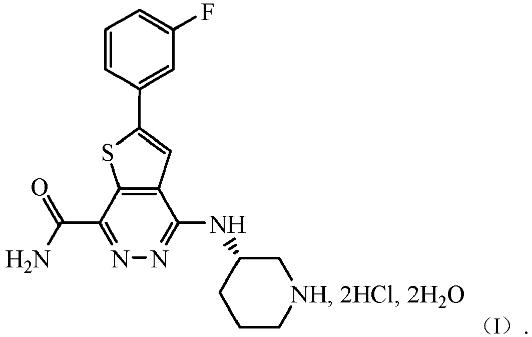

2. The crystal form of thiofuran pyridazine compound according to claim 1, wherein the molar ratio of (S)-2-(4-fluorophenyl)-4-(piperidine-3-aminomethyl)thieno[2,3-d] pyridinazine -7-carboxamide to hydrochloric acid is 1:2.

3. The crystal form of thiofuran pyridazine compound according to claim 2, wherein further comprises two molecules of water.

4. The crystal form of thiofuran pyridazine compound according to any one of claims 1-3, wherein a X-ray powder diffraction spectrum expressed by CuKα radiation and 2θ shows the diffraction peaks are at least at 4.11±0.20°, 5.82±0.20°, 6.85±0.20°, 7.41±0.20°, 8.22±0.20°, 9.67±0.20°, 13.73±0.20°, 14.10±0.20°, 14.85±0.20°.

5. A preparation method of the crystal form of thiofuran pyridazine compound according to any one of claims 1-4, wherein (S)-2-(4-fluorophenyl)-4-(piperidine-3-aminomethyl)thieno [2,3-d]pyridazine-7-carboxamide reacts with hydrochloric acid dissolved in an aqueous polar solvent, to obtain (S)-2-(4-fluorophenyl)-4-(piperidine-3-aminomethyl)thieno[2,3-d] pyridazine-7-carboxamide hydrochloride.

6. The preparation method according to claim 5, wherein the molar ratio of (S)-2-(4-fluorophenyl)-4-(piperidine-3-aminomethyl)thieno[2,3-d]pyridazine-7-carboxamide to hydrochloric acid is 1:2.

7. The preparation method according to claim 5, wherein the polar solvent is at least selected from one of C1~C4 monohydric alcohol, acetonitrile, acetone.

8. The preparation method according to claim 5, wherein the volume ratio of the polar solvent to water is 1~ 5: 1.

9. An application of the crystal form of thiofuran pyridazine compound according to any one of claims 1-4 in preparation for pharmaceuticals of treatment and prevention of tumour.
